Europäisches Patentamt

European Patent Office

Office européen des brevets

(19)

(11) **EP 0 489 662 B1**

(12)

# FASCICULE DE BREVET EUROPEEN

(45) Date de publication et mention
de la délivrance du brevet:
**04.09.1996 Bulletin 1996/36**

(51) Int Cl.⁶: **G01N 11/04**, G01N 33/20

(21) Numéro de dépôt: **91403303.0**

(22) Date de dépôt: **06.12.1991**

(54) **Procédé et dispositif de mesure des caractéristiques rhéologiques d'un matériau à une température déterminée**

Vorrichtung und Verfahren zur Messung der rheologischen Eigenschaften eines Materials bei einer bestimmten Temperatur

Method and device for determining the rheological properties of a material at a given temperature

(84) Etats contractants désignés:
**AT BE CH DE DK ES FR GB GR IT LI LU NL SE**

(30) Priorité: **06.12.1990 FR 9015308**

(43) Date de publication de la demande:
**10.06.1992 Bulletin 1992/24**

(73) Titulaire: **ASCOMETAL**
**F-92072 PARIS-LA DEFENSE CEDEX 35 (FR)**

(72) Inventeurs:
- **Secordel, Pascal**
  **F-43110 Aurec sur Loire (FR)**
- **Valette, Elisabeth**
  **F-42490 Fraisses (FR)**
- **Leroy, François**
  **F-42700 Firminy (FR)**
- **Branswyck, Olivier**
  **F-06700 St. Laurent du Var (FR)**
- **Levaillant, Christophe**
  **F-06560 Valbonne (FR)**

(74) Mandataire: **Lanceplaine, Jean-Claude et al**
**CABINET LAVOIX**
**2, Place d'Estienne d'Orves**
**75441 Paris Cédex 09 (FR)**

(56) Documents cités:
**EP-A- 0 210 689        FR-A- 2 369 558**
**US-A- 3 203 225**

- **METALS TECHNOLOGY vol. 9, Septembre 1982, GB, pp. 360-367; K.E. HUGHES et al.: "Influence of reheating temperature 680-1280 C on extrusion of mild steel"**
- **THE METALLUGIST & MATERIALS TECHNOLOGIST vol. 5, Novembre 1973, GB, pp. 572-578; K.E. HUGHES : "Some factors affecting the hot extrusion of steel"**

EP 0 489 662 B1

# Description

La présente invention concerne un procédé de mesure des caractéristiques rhéologiques d'un matériau à une température déterminée, dans lequel on analyse les comportements rhéologiques d'un matériau tel qu'un métal et notamment d'un acier, à l'état semi-solide à la température de la mesure.

La présente invention concerne également un dispositif pour la mise en oeuvre de ce procédé.

Différents procédés et dispositifs connus sont utilisés pour des caractérisations rhéologiques.

Les viscomètres type Couette, permettent, par mesure d'un couple résistant, de déterminer une viscosité ou une viscosité apparente en fonction de la vitesse de cisaillement.

Les viscomètres cône - plan, présentent l'avantage, par rapport à la technique précédente, de générer dans le matériau une vitesse de cisaillement uniforme.

Ces techniques imposent l'établissement d'un régime permanent d'écoulement, la nécessité d'utiliser un matériau homogène, et sont limitées à des cisaillement purs avec des vitesses de cisaillement élevées comprises entre 100 et 1000 s$^{-1}$. Dans l'application aux mesures rhéologiques d'un acier, il est nécessaire d'utiliser un mélange liquide - solide homogène, sans évolution rapide de la fraction volumique de la phase solide ou de la morphologie des grains solides. Cette condition impose en particulier de travailler sur un métal semi-solide obtenu par refroidissement contrôlé à partir de l'état liquide. Enfin, ces techniques connues ne permettent pas de faire varier de façon indépendante les champs de déformation et de vitesse de déformation ou de vitesse de cisaillement.

Dans un autre procédé de mesure connu, on réalise un essai de compression entre plateaux parallèles sous charge constante.

Cette technique, se rapprochant d'une opération de mise en forme, permet de travailler sur des échantillons refondus partiellement à partir de l'état solide. Pour ces raisons technologiques, on est, dans ce procédé connu, limité à des vitesses de cisaillement faiblesse de l'ordre de $(10^{-5}, 10^{-2})$s$^{-1}$, et à des déformations limitées ($\varepsilon <$ 1,5).

On connaît dans le document FR-A-2 369 558 un procédé destiné à déterminer les propriétés des matériaux extrudables et, en particulier, à déterminer l'aptitude au traitement des élastomères. Le procédé consiste à préchauffer dans un four un lopin solide en élastomère, à déplacer le lopin préchauffé dans une filière prévue à une extrémité d'une chambre longitudinale, à chauffer le lopin à une température déterminée dans la chambre longitudinale, à comprimer le lopin dans la chambre longitudinale et à déformer le lopin par filage, puis à mesurer les caractéristiques rhéologiques de la déformation isotherme de l'élastomère pendant le filage à la température déterminée.

On connaît également dans le brevet EP-A-0 210 689 un appareillage pour la détermination des propriétés rhéologiques d'une matière thermoplastique comprenant un boîtier de commande et de contrôle, un cylindre muni d'une filière et destiné à recevoir une dose déterminée de la matière thermoplastique à évaluer, un piston pouvant coulisser dans le cylindre, un moteur à commande pas à pas assurant le déplacement du piston, un capteur disposé entre le moteur et le piston et un système électronique à microprocesseur contrôlant le moteur en fonction de la position du piston et/ou du signal émis par le capteur.

Par ailleurs, l'article "METALS TECHNOLOGY" Vol. 9, September 1982, GB - pages 360 - 367 HUGHES K. E. et AI montre l'influence de la température sur l'extrusion de l'acier. Dans cet article on effectue un examen métallographique des échantillons après extrusion et une corrélation des résultats obtenus avec des mesures mécaniques.

Le but de la présente invention est d'étudier le comportement rhéologique d'un matériau tel qu'un acier semi-solide, de déterminer les paramètres de la loi de comportement et d'apprécier la sollicitation comme par exemple la température, la vitesse de déformation, le taux de déformation.

La présente invention a pour objet un procédé de mesure des caractéristiques rhéologiques d'un métal ou d'un alliage métallique à une température déterminée, caractérisé en ce que :

- on préchauffe un lopin solide du métal ou de l'alliage métallique à analyser dans un four,
- on déplace le lopin préchauffé dans une chambre longitudinale d'une filière comportant à une extrémité une paroi munie d'un trou de filière,
- on chauffe le lopin dans la chambre longitudinale pour lui conférer un état semi-solide, à une température comprise entre la température de solidus et la température de liquidus,
- on compresse le lopin dans ladite chambre,
- on déforme le lopin par filage, le métal ou l'alliage métallique déformé passant par le trou de filière,
- on mesure les caractéristiques rhéologiques de la déformation isotherme du métal ou de l'alliage métallique pendant le filage à la température déterminée,
- on caractérise la structure du métal ou de l'alliage métallique déformé, après filage, à la température ambiante,
- et on corrèle la déformabilité du métal ou de l'alliage métallique après déformation, puis refroidissement à la température ambiante, soit aux données métallurgiques, soit aux données mécaniques.

Selon d'autres caractéristiques de l'invention :

- on modélise la déformation par une méthode numérique, en affectant aux paramètres de la loi de comportement du métal ou de l'alliage métallique, des valeurs arbitraires admissibles,

- on établit, à partir de cette modélisation, des courbes théoriques servant à la construction d'abaques auxquels seront comparées les courbes déterminées expérimentalement,
- on identifie les paramètres rhéologiques réels du métal ou de l'alliage métallique analysé en portant dans ces abaques les courbes expérimentales,
- on établit, à partir de cette modélisation, les champs de déformation, de vitesse de déformation et de pression hydrostatique dans le métal ou l'alliage métallique au cours de la mesure,
- dans la modélisation numérique de mesure on introduit les caractéristiques d'un métal ou d'un alliage métallique ayant un comportement rhéologique représentatif particulier de référence,
- dans la modélisation numérique de mesure on introduit les caractéristiques du métal ou de l'alliage métallique biphasé à un comportement de type viscoplastique non écrouissable d'un métal ou d'un alliage métallique présentant une loi de comportement du type viscoélastique compressible,
- on contrôle la température de refroidissement métal ou de l'alliage métallique mis en forme, par filage, en sortie de la filière,
- on mesure les caractéristiques rhéologiques d'un métal ou d'un alliage métallique biphasé,
- on effectue les mesures des vitesses de cisaillement comprises dans l'intervalle $(10^{-5}, 10^3)s^{-1}$,
- on compresse le lopin dans la chambre longitudinale par déplacement d'un piston formant avec la chambre une pompe de compression,
- on compresse le lopin dans la chambre longitudinale par déplacement d'une filière formant avec la chambre une pompe de compression,
- on compresse le lopin dans la chambre longitudinale par déplacement d'un piston formant avec la chambre une pompe de compression, le métal ou l'alliage métallique déformé s'écoulant en périphérie dudit piston.

La présente invention concerne également un dispositif pour la mise en oeuvre du procédé, comprenant une filière composée d'une chambre longitudinale et d'une paroi comportant un trou de filière en céramique, un suscepteur pour le chauffage de la filière et du lopin formant un support mécanique des efforts transmis à la filière lors du filage du lopin, des moyens de chauffage du suscepteur, des moyens de mesure en température de la filière et du lopin logé dans la chambre, un four de préchauffe placé en amont de la filière pour le préchauffage du lopin, des moyens de déplacement longitudinal du lopin, du four de préchauffe vers la chambre de filière, et des moyens de pression pour déformer le lopin par filage à travers le trou de filière.

Selon d'autre caractéristiques de l'invention:

- la filière est en alumine,

- la structure est à base de molybdène, pour en supporter les efforts mécaniques de filage à des températures supérieures à 1500°C,
- le dispositif comprend, en outre, une enceinte de refroidissement contrôlée du matériau après la mise en forme par filage du lopin,
- les moyens de déplacement et de pression sont constitués par un piston coulissant dans la chambre longitudinale de la filière pour former une pompe de compression.

D'autres caractéristiques et avantages de l'invention appraraîtront au cours de la description qui va suivre, faite en se référant aux dessins annexés donnés uniquement à titre d'exemple et sur lesquels :

- la Fig. 1 est une vue en coupe longitudinale du dispositif de mesure selon l'invention,
- la Fig. 2 est un abaque établi en comparant les efforts stabilisés du filage en cours de mesures réalisés à différentes vitesses de filage,
- la Fig. 3 donne une carte des isovaleurs de vitesse de déformation généralisées dans le cas d'une mesure de filage direct isotherme,
- la Fig. 4 donne une carte des isovaleurs de densité dans le cas d'un essai de filage direct isotherme d'un matériau biphasé compressible,
- la Fig. 5 montre un exemple de structure obtenue par filage directe isotherme d'un acierr à 1% de carbone et 1,4% de chrome.

Le procédé selon l'invention consiste en l'utilisation conjointe, pour déterminer les caractéristiques rhéologiques d'un matériau semi-solide, d'un essai de filage isotherme et de sa modélisation par une méthode numérique.

Pour cela, on préchauffe un lopin solide d'un matériau, comme par exemple un acier. Le préchauffage est réalisé dans un four de préchauffe en amont de la filière, pour réduire le temps de chauffage du lopin. Les moyens de chauffage du four de préchauffe sont plus confinés autour du lopin que les éléments de chauffage de la filière.

On déplace le lopin d'acier, lorsqu'il est à une température supérieure à 1280°C et dans cet exemple de réalisation à environ 1350°C, dans une chambre longitudinale de la filière, afin d'ajuster la température du lopin pour la mesure de rhéologie. On compresse le lopin dans la chambre de la filière, par exemple, au moyen d'un piston, la chambre et le piston formant une pompe qui pousse le métal semi-solide pour l'éjecter par le trou de filière.

Lors du filage, la mesure de rhéologie consiste :

- à enregistrer l'effort de filage en fonction du déplacement du piston, avec les paramètres opératoires qui sont la température, la vitesse de filage et le taux de filage.

- à tracer des abaques théoriques : efforts de filage - déplacements, à partir des résultats de modélisations numériques réalisées en affectant aux paramètres de la loi de comportement, des valeurs arbitraires admissibles,
- à comparer les résultats théoriques et les résultats expérimentaux.

Par ailleurs, on modèlise la déformation par une méthode numérique, en affectant aux paramètres de la loi de comportement du matériau, des valeurs arbitraires admissibles et on établit, à partir de cette modélisation, des courbes théoriques servant à la construction d'abaques auxquels seront comparées les courbes déterminées expérimentalement.

Ensuite, d'une part, on identifie les paramètres rhéologiques réels du matériau analysé en portant dans ces abaques les courbes expérimentales et, d'autre part, on établit à partir de cette modélisation, les champs de déformation, de vitesses de déformation et de pression hydrostatique dans le matériau au cours de la mesure.

Dans la modélisation numérique de mesure, on introduit les caractéristiques d'un matériau ayant un comportaient rhéologique représentatif particulier de référence comme par exemple un comportement de type viscoplastique non écrouissable, ou d'un matériau biphasé à comportement de type compressible, l'une des deux phases présentant un comportement de type viscoplastique non écrouissable.

Les vitesses de déformation dans le métal dépendent de la vitesse de filage et du taux de filage.

La température d'essai comprise entre la température de solidus et de liquidus fixe la fraction volumique de phase solide dans l'alliage au cours de d'essai.

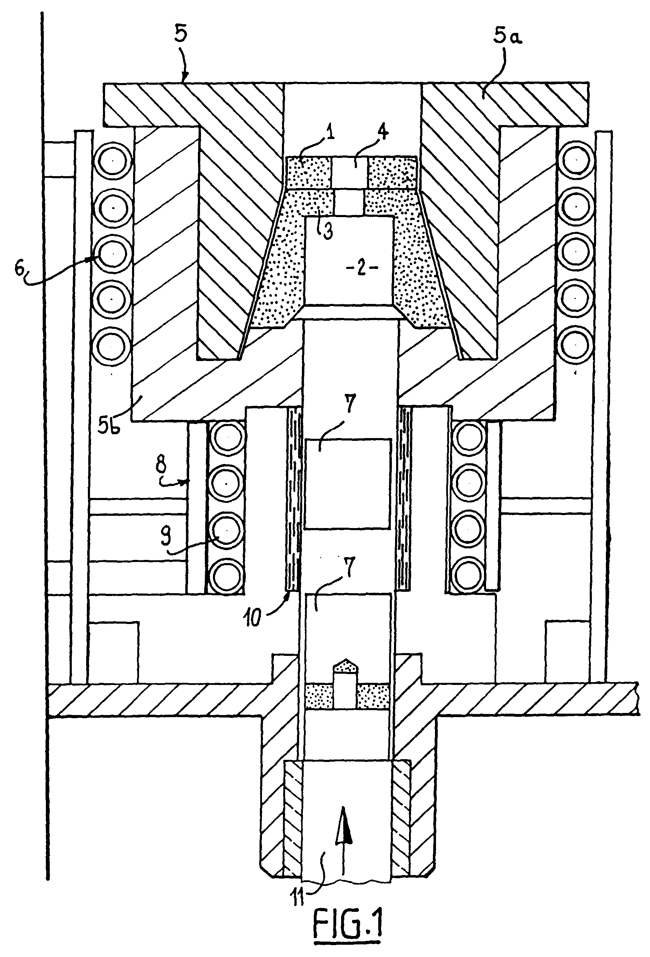

La figure 1 représente un dispositif de mesure de la rhéologie d'un matériau comme par exemple, d'un acier à l'état semi-solide.

Le dispositif comprend une filière 1 en matériau céramique composée d'une chambre longitudinale 2 et d'une paroi 3 comportant un trou 4 de filière. La filière 1 par exemple en alumine est disposée dans un suscepteur 5 en alliage à base molybdène et positionné sur un support 5b en céramique isolante.

Le suscepteur 5 de filière maintient mécaniquement la filière 1 soumise à des efforts mécaniques dus au métal semi-solide qui est lui même soumis à une compression lors de la mesure.

Le support du suscepteur 5 et de la filière 1 comprend des moyens de chauffage 6 du suscepteur 5 qui par conduction et convection permet le chauffage de la chambre 2 et d'un lopin 7 introduit dans ladite chambre. Ces moyens de chauffage 6 sont, dans cet exemple de réalisation, constitués d'un inducteur alimenté en courant alternatif et permettant le chauffage du suscepteur 5 de filière.

La filière 1 et le suscepteur 5 de filière sont contrôlés en température par des moyens de mesure, tels que par exemple des thermocouples, et/ou des pyromètres, non représentes sur la figure 1.

En amont de la filière 1 et du suscepteur 5 de filière, le dispositif comporte un four de préchauffe 8 du lopin 7, longitudinal, placé coaxialement à la chambre 2 de filière. Ce four 8 comporte des moyens de chauffage constitués également d'un inducteur 9 entourant un tube 10 de quartz. Le lopin 7 est placé coaxialement au four de préchauffe 8 et à la chambre 2 de filière.

Le dispositif comporte également un moyen de pression pour le filage direct isotherme du lopin 7, qui est dans, cet exemple, constitué par un piston 11 formant avec la chambre 2 de filière une pompe de compression du métal contenu dans ladite chambre. D'autre part, le piston 11 est un moyen de déplacement du lopin 7, du four de préchauffe 8 vers la chambre 2 de filière.

Le dispositif peut comporter, à la sortie du trou 4 de filière une enceinte de refroidissement, non représentée, qui contrôle en température, le métal déformé après filage isotherme.

Selon une variante, le lopin 7 peut être compressé dans la chambre longitudinale 2 par déplacement d'une filière formant avec ladite chambre une pompe de compression.

Selon encore une autre variante, le lopin 7 peut être compressé dans la chambre longitudinale 2 par déplacement d'un piston formant avec ladite chambre une pompe de compression, le matériau déformé s'écoulant en périphérie dudit piston.

Le procédé et le dispositif selon l'invention permettent :

- de réaliser un essai rhéologique proche d'un essai réel de mise en forme, permettant de provoquer un écoulement du métal semi-solide,
- de réaliser un essai rhéologique mettant en oeuvre des champs de déformations et de vitesses de déformations généralisées, permettant une phase solide pouvant être indifféremment de morphologie dendritique ou globulaire et non réduites à des cisaillements et des vitesses de cisaillement purs,
- de faire varier dans une large gamme, les vitesses de déformation, et en particulier de couvrir les vitesses comprises entre $10^{-5}$ et $10^3$ s$^{-1}$, et de préférence comprises entre $10^{-2}$ et $10^2$ s$^{-1}$, en faisant varier le taux et/ou la vitesse de filage,
- de faire varier les taux de déformation du métal en modifiant le rapport de filage,
- d'apprécier, outre les paramètres rhéologiques de la loi de comportement, la déformabilité de l'acier semi-solide, c'est-à-dire, sa capacité à s'écouler de manière homogène, et de la relier à des données métallurgiques telles que taille du grain, fraction de phase solide ou mécanique comme par exemple les champs de pression, de déformation et de vitesses de déformation.

Dans le cas où l'on suppose par exemple une loi de comportement du type viscoplastique, les paramètres rhéologiques sont la consistance, ainsi que le coefficient de sensibilité à la vitesse de déformation définis par la relation suivante :

$$[S] = 2.K \left( \sqrt{3} \; \dot{\overline{\varepsilon}} \right)^{m-1} . [\dot{\varepsilon}]$$

dans laquelle

[S]     est le tenseur déviateur des contraintes

K     la consistance en MPa $s^m$

$\dot{\overline{\varepsilon}}$     la vitesse de déformation généralisée

$[\dot{\varepsilon}]$     le tenseur des vitesses de déformation

m     le coefficient de sensibilité à la vitesse de déformation

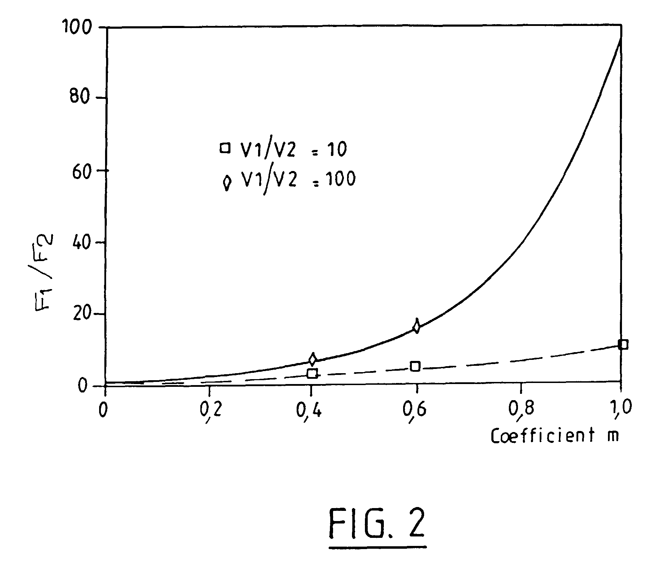

L'abaque de la figure 2 est établi en comparant les efforts stabilisés de filage, en cours d'essais, réalisés à différentes vitesses de filage. Pour un alliage donné, à une température fixée, le rapport F1/F2 des efforts stabilisés obtenus aux vitesses V1 et V2 est unique, pour une valeur fixe de la consistance K et correspond à une valeur du coëfficient m de sensibilité à la vitesse de déformation.

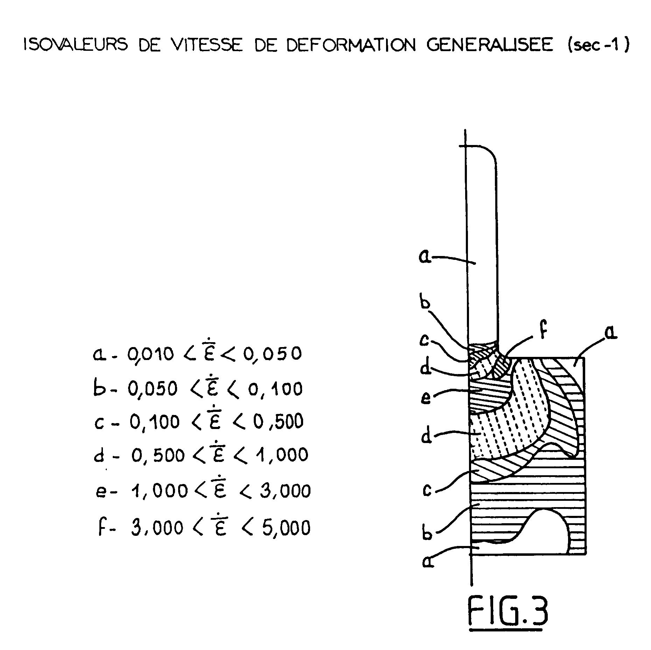

La figure 3 donne une carte des isovaleurs de vitesses de déformations généralisées dans le cas d'un essai de filage direct isotherme réalisé sur un acier de construction présentant environ 50% de phase solide semi globulaire, avec une vitesse de filage 0,4mm/s et un rapport de filage de 14. Les coefficients rhéologiques admissibles sont 1 MPas$^m$ et 0,2 respectivement pour la consistance K et le coefficient m de sensibilité à la vitesse de déformation. Dans ces conditions de filage, et pour la géométrie de filière utilisée, les valeurs des vitesses de déformation généralisées sont comprises entre $10^{-2}s^{-1}$ et $6s^{-1}$.

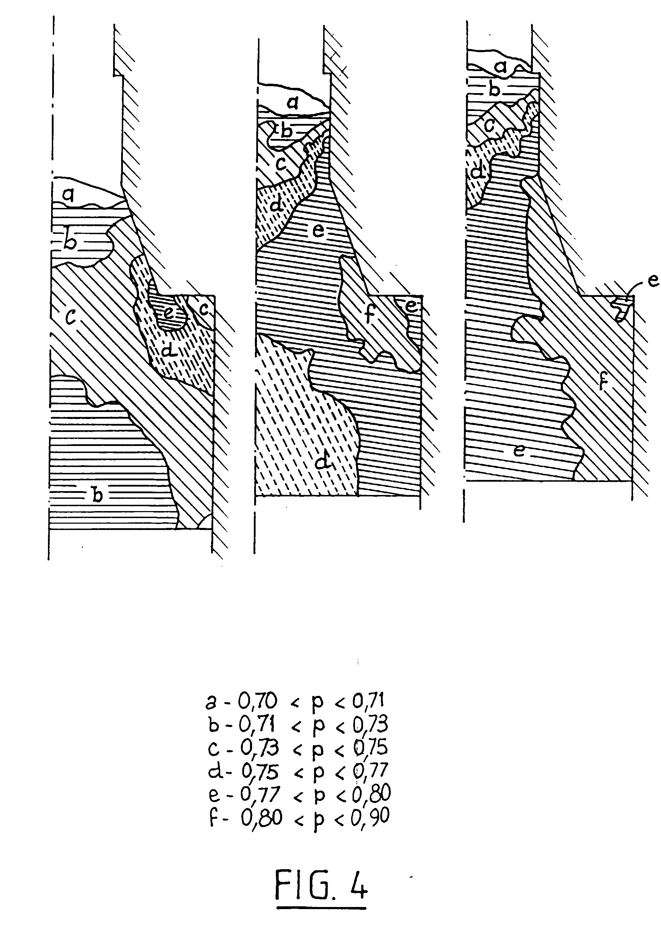

La figure 4 donne une carte des isovaleurs de densité dans le cas d'un essai de filage direct isotherme réalisé sur un acier de construction présentant initialement 70% environ de phase solide, avec une vitesse de filage de 4mn/s, et un rapport de filage de 5. Les coefficients rhéologiques admissibles sont 1 MPas$^m$ et 0.2 respectivement pour la consistance K et le coefficient m de sensiblitité à la vitesse de déformation de la phase solide.

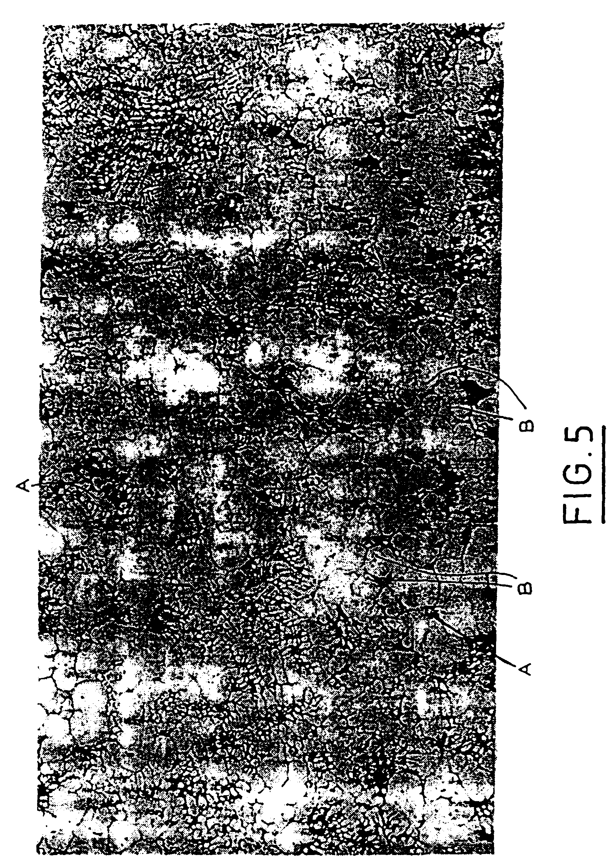

Un exemple de structure observée dans un fil obtenu par filage direct isotherme à 1400°C d'un acier à 1%C, 1,4%Cr est donné à la figure 5. Le refroidissement contrôlé en sortie de filière permet de différencier la phase liquide au cours du filage, en ce qu'elle se présente, après refroidissement, sous forme de dendrites fines A, de la phase solide au cours du filage, apparaissant sous forme de particules semi-globulaires B. On visualise ainsi les écoulements relatifs liquide/solide au cours du filage.

**Revendications**

1.  Procédé de mesure des caractéristiques rhéologiques d'un métal ou d'un alliage métallique à une température déterminée, caractérisé en ce que :

    -   on préchauffe un lopin (7) solide du métal ou de l'alliage métallique à analyser dans un four (8),
    -   on déplace le lopin (7) préchauffé dans une chambre longitudinale (2) d'une filière (1) comportant à une extrémité une paroi (3) munie d'un trou (4) de filière,
    -   - on chauffe le lopin (7) dans la chambre longitudinale (2) pour lui conférer un état semi-solide, à une température comprise entre la température de solidus et la température de liquidus,
    -   on compresse le lopin (7) dans ladite chambre longitudinal (2),
    -   on déforme le lopin (7) par filage, le métal ou l'alliage métallique déformé passant dans le trou (4) de filière,
    -   on mesure les caractéristiques rhéologiques de la déformation isotherme du métal ou de l'alliage métallique pendant le filage à la température déterminée,
    -   on caractérise la structure du métal ou de l'alliage métallique déformé, après filage, à la température ambiante,
    -   et on corrèle la déformabilité du métal ou de l'alliage métallique après déformation, puis refroidissement à la température ambiante, soit aux données métallurgiques, soit aux données mécaniques.

2.  Procédé selon la revendication 1, caractérisé en ce que :

    -   on modélise la déformation par une méthode numérique, en affectant au paramètre de la loi de comportement du métal ou de l'alliage métallique des valeurs arbitraires admissibles,

    -   on établit, à partir de cette modélisation, des courbes théoriques servant à la construction d'abaques auxquels seront comparées les courbes déterminées expérimentalement,

    -   on identifie les paramètres rhéologiques réels du métal ou de l'alliage métallique analysé en portant dans ces abaques les courbes expérimentales,

    -   on établit, à partir de cette modélisation, les champs de déformation, de vitesse de déformation et de pression hydrostatique dans le métal ou l'alliage métallique au cours de la mesure.

3. Procédé selon les revendications 1 et 2, caractérisé en ce que, dans la modélisation numérique de mesure, on introduit les caractéristiques d'un métal ou d'un alliage métallique biphasé ayant un comportement rhéologique représentatif particulier de référence.

4. Procédé selon l'une quelconque des revendications 1 à 3, caractérisé en ce que, dans la modélisation numérique de mesure, on introduit les caractéristiques rhéologiques du métal ou de l'alliage métallique biphasé à comportement de typé viscoplastique non écrouissable ou d'un métal ou d'un alliage métallique présentant une loi de comportement du type viscoélastique compressible.

5. Procédé selon l'une quelconque des revendications précédentes, caractérisé en ce que l'on contrôle la température du refroidissement du métal ou de l'alliage métallique mis en forme par filage, en sortie de la filière.

6. Procédé selon l'une quelconque des revendications précédentes, caractérisé en ce que l'on mesure les caractéristiques rhéologiques d'un métal ou d'un alliage métallique biphasé.

7. Procédé selon l'une quelconque des revendications précédentes, caractérisé en ce que l'on effectue les mesures à des vitesses de cisaillement comprises dans l'intervalle $(10^{-5}, 10^3)s^{-1}$.

8. Procédé selon l'une quelconque des revendications précédentes, caractérisé en ce que l'on compresse le lopin (7) dans la chambre longitudinale (2) par déplacement d'un piston (11) formant avec la chambre (2) une pompe de compression.

9. Procédé selon l'une quelconque des revendication 1 à 7, caractérisé en ce que l'on compresse le lopin (7) dans la chambre longitudinale (2) par déplacement d'une filière formant avec la chambre (2) une pompe de compression.

10. Procédé selon l'une quelconque des revendications 1 à 7, caractérisé en ce que l'on compresse le lopin (7) dans la chambre longitudinale (2) par déplacement d'un piston formant avec la chambre (2) une pompe de compression, le métal ou l'alliage métallique déformé s'écoulant en périphérie dudit piston.

11. Dispositif de mesures des caractéristiques rhéologiques d'un métal ou d'un alliage métallique à une température déterminée pour la mise en oeuvre du procédé selon l'une quelconque des revendications 1 à 10, caractérisé en ce qu'il comprend une filière (1) composée d'une chambre longitudinale (2) et d'une paroi (3) comportant un trou (4) de filière en céramique, un suscepteur (5) pour le chauffage de la filière (1) et du lopin (2) formant un support mécanique des efforts transmis à la filière (1) lors du filage du lopin (7), des moyens de chauffage (6) du suscepteur (5), des moyens de mesure en température de la filière (1) et du lopin (7) logé dans la chambre (2), un four de préchauffe (8) placé en amont de la filière (1) pour le préchauffage du lopin (7), des moyens de déplacement longitudinal du lopin (7), du four de préchauffe (8) vers la chambre (2) de la filière et des moyens de pression pour déformer le lopin (7) par filage à travers le trou (4) de filière.

12. Dispositif selon la revendication 11, caractérisé en ce que la filière (1) est en alumine.

13. Dispositif selon la revendication 11, caractérisé en ce que le suscepteur (5) est à base de molybdène, pour en supporter les efforts mécaniques de filage à des températures supérieures à 1500°C.

14. Dispositif selon la revendication 11, caractérisé en ce qu'il comprend en outre, une enceinte de refroidissement contrôlée du matériau après mise en forme par filage du lopin (7).

15. Dispositif selon la revendication 11, caractérisé en ce que les moyens de déplacement de pression sont constitués par un piston (11) coulissant dans la chambre longitudinale (2) de la filière (1) pour former une pompe de compression.

**Patentansprüche**

1. Verfahren zur Messung der rheologischen Eigenschaften eines Metalls oder einer Metallegierung bei einer bestimmten Temperatur, dadurch gekennzeichnet, daß:

- ein fester Rohling (7) des zu analysierenden Metalls oder der Metallegierung in einem Ofen (8) vorerhitzt wird;
- der vorerhitzte Rohling (7) in eine Längskammer (2) einer Matrize (1) gebracht wird, die an einem Ende eine Wand (3) umfaßt, die mit einer Matrizenöffnung (4) versehen ist;
- der Rohling (7) in der Längskammer (2) erhitzt wird, um ihm einen halb-festen Zustand zu verleihen, bei einer Temperatur, die zwischen der Temperatur des festen und jener des flüssigen Zustandes liegt;

- der Rohling (7) in dieser Längskammer (2) zusammengedrückt wird;

- der Rohling (7) durch Fließpressen verformt wird, wobei das verformte Metall oder die verformte Metallegierung durch die Matrizenöffnung (4) fließt;

- die rheologischen Eigenschaften der isothermischen Verformung des Metalls oder der Metallegierung während des Fließpressens bei der bestimmten Temperatur gemessen werden;

- die Struktur des verformten Metalls oder der Metallegierung nach dem Fließpressen bei Raumtemperatur charakterisiert wird;

- und die Verformbarkeit des Metalls oder der Metallegierung nach Verformung und sodann Abkühlung bei Raumtemperatur entweder mit den metallurgischen Daten oder mit den mechanischen Daten in Korrelation gebracht wird.

2. Verfahren nach Anspruch 1, dadurch gekennzeichnet, daß

- die Verformung durch eine numerische Methode gestaltet wird, indem dem Parameter des Verhaltensgesetzes des Metalls oder der Metallegierung willkürliche zulässige Werte zugeordnet werden;

- ausgehend von dieser Gestaltung theoretische Kurven erstellt werden, die zur Erstellung von Schaulinientafeln dienen, mit denen die experimentell bestimmten Kurven verglichen werden;

- die tatsächlichen rheologischen Parameter des analysierten Metalls oder der Metallegierung festgestellt werden, indem in diese Schaulinientafeln die experimentellen Kurven eingetragen werden;

- anhand dieser Gestaltung die Bereiche der Verformung, der Verformungsgeschwindigkeit und des hydrostatischen Drucks im Metall oder in der Metallegierung während der Messung erstellt werden.

3. Verfahren nach einem der Ansprüche 1 und 2, dadurch gekennzeichnet, daß bei der numerischen Meßgestaltung die Eigenschaften eines zweiphasigen Metalls oder einer zweiphasigen Metallegierung, die ein repräsentatives, besonderes rheologisches Referenzverhalten aufweisen, eingeführt werden.

4. Verfahren nach einem der Ansprüche 1 bis 3, dadurch gekennzeichnet, daß bei der numerischen Meßgestaltung die rheologischen Eigenschaften des zweiphasigen Metalls oder der Metallegierung mit viskos-plastischem, nicht kaltverformbarem Verhalten oder eines Metalls oder einer Metallegierung eingeführt werden, die ein zusammendrückbares, viskos-elastisches Verhaltensgesetz aufweisen.

5. Verfahren nach einem der vorhergehenden Ansprüche, dadurch gekennzeichnet, daß die Kühltemperatur des Metalls oder der Metallegierung, die durch Fließpressen geformt wurde, am Matrizenausgang gemessen wird.

6. Verfahren nach einem der vorhergehenden Ansprüche, dadurch gekennzeichnet, daß die rheologischen Eigenschaften eines zweiphasigen Metalls oder einer zweiphasigen Metallegierung gemessen werden.

7. Verfahren nach einem der vorhergehenden Ansprüche, dadurch gekennzeichnet, daß die Messungen bei Schergeschwindigkeiten im Bereich $(10^{-5}, 10^3)$ $s^{-1}$ durchgeführt werden.

8. Verfahren nach einem der vorhergehenden Ansprüche, dadurch gekennzeichnet, daß der Rohling (7) in der Längskammer (2) durch Verschieben eines Kolbens (11), der mit der Kammer (2) eine Preßpumpe bildet, zusammengedrückt wird.

9. Verfahren nach einem der Ansprüche 1 bis 7, dadurch gekennzeichnet, daß der Rohling (7) in der Längskammer (2) durch Verschieben einer Matrize, die mit der Kammer (2) eine Preßpumpe bildet, zusammengedrückt wird.

10. Verfahren nach einem der Ansprüche 1 bis 7, dadurch gekennzeichnet, daß der Rohling (7) in der Längskammer (2) durch Verschieben eines Kolbens, der mit der Kammer (2) eine Preßpumpe bildet, zusammengedrückt wird, wobei das verformte Metall oder die verformte Metallegierung an der Peripherie dieses Kolbens abfließt.

11. Vorrichtung zur Messung der rheologischen Eigenschaften eines Metalls oder einer Metallegierung bei einer bestimmten Temperatur, die das erfindungsgemäße Verfahren nach einem der Ansprüche 1 bis 10 einsetzt, dadurch gekennzeichnet, daß sie eine Matrize (1) umfaßt, bestehend aus einer Längskammer (2) und einer Wand (3), bestehend aus einer Matrizenöffnung (4) aus Keramik, einer Leitvorrichtung (5) für die Erhitzung der Matrize (1) und des Rohlings (2), die eine mechanische Stütze für die bei dem Fließpressen des Rohlings (7) auf die Matrize (1) übertragenen Kräfte bildet, Mitteln zum Erhitzen (6) der Leitvorrichtung (5), Mitteln zum Messen der Temperatur der Matrize (1) und des in der Kammer (2) befindlichen Rohlings (7), einem Vorheizofen (8), der im Vorlaufbereich der Matrize (1) für die Vorerhitzung des Rohlings (7) angeordnet ist, Mitteln zur Längsverschiebung des Rohlings (7) vom Vorheizofen (8) zu der Kammer (2) der Matrize und Druckmitteln zur Verformung des Rohlings (7) durch Fließpressen durch die Matrizenöffnung (4).

12. Vorrichtung nach Anspruch 11, dadurch gekennzeichnet, daß die Matrize (1) aus Aluminiumoxid besteht.

13. Vorrichtung nach Anspruch 11, dadurch gekennzeichnet, daß die Leitvorrichtung (5) auf Basis von Molybdän hergestellt ist, um den mechanischen Kräften des Fließpressens bei Temperaturen von über 1500°C standzuhalten.

14. Vorrichtung nach Anspruch 11, dadurch gekennzeichnet, daß sie ferner eine kontrollierte Kühlzelle des Materials nach Formung des Rohlings (7) durch Fließpressen umfaßt.

15. Vorrichtung nach Anspruch 11, dadurch gekennzeichnet, daß die Druckverschiebemittel von einem Kolben (11) gebildet werden, der in der Längskammer (2) der Matrize (1) gleitet, um eine Preßpumpe zu bilden.

## Claims

1. Process for measuring the rheological characteristics of a metal or metal alloy at a specified temperature, characterised in that:

   a solid slab (7) of the metal or metal alloy to be analysed is preheated in a furnace (8),
   the preheated slab (7) is moved into a longitudinal chamber (2) of a die plate (1) comprising, at one end, a wall (3) provided with a die (4),
   the slab (7) is heated in the longitudinal chamber (2) in order to convert it into a semi-solid state, at a temperature between the temperature of the solidus and the temperature of the liquidus,
   the slab (7) is compressed in said longitudinal chamber (2),
   the slab (7) is deformed by drawing, the deformed metal or metal alloy passing into the die (4),
   the rheological characteristics of the isothermic deformation of the metal or metal alloy are measured during drawing at the specified temperature,
   the structure of the deformed metal or metal alloy after drawing is characterised at ambient temperature,
   and the deformability of the metal or metal alloy after deformation followed by cooling to ambient temperature is correlated either with the metallurgical data or with the mechanical data.

2. Process according to claim 1, characterised in that:

   the deformation is modelled by a numerical method, by assigning arbitrary permissible values to the parameter of the law of behaviour of the metal or metal alloy,
   starting from this model, theoretical curves are plotted which are used to draw up charts with which the experimentally determined curves will be compared,
   the actual rheological parameters of the metal or metal alloy analysed are identified by entering the experimental curves in these charts,
   starting from this model, the deformation ranges, rate of deformation and hydrostatic pressure in the metal or metal alloy being measured are established.

3. Process according to claims 1 and 2, characterised in that the characteristics of a metal or a two-phase metal alloy having a particular representative rheological behaviour are entered into the numerical measuring model to act as reference.

4. Process according to any one of claims 1 to 3, characterised in that the rheological characteristics of the metal or two-phase metal alloy having a non-hammer-hardenable viscoplastic behaviour or a metal or metal alloy having compressible viscoelastic-type behavioural characteristics are entered into the numerical measuring model.

5. Process according to any one of the preceding claims, characterised in that the cooling temperature of the metal or metal alloy shaped by drawing is monitored at the exit from the die plate.

6. Process according to any one of the preceding claims, characterised in that the rheological characteristics of a metal or a two-phase metal alloy are measured.

7. Process according to any one of the preceding claims, characterised in that the measurements are taken at shear velocities within the range ($10^{-5}$, $10^{3}$) $s^{-1}$.

8. Process according to any one of the preceding claims, characterised in that the slab (7) is compressed in the longitudinal chamber (2) by the movement of a piston (11) forming a compression pump with the chamber

9. Process according to any one of claims 1 to 7, characterised in that the slab (7) is compressed in the longitudinal chamber (2) by the displacement of a die plate forming a compression pump with the chamber (2).

10. Process according to any one of claims 1 to 7, characterised in that the slab (7) is compressed in the longitudinal chamber (2) by the movement of a piston forming a compression pump with the chamber (2), the deformed metal or metal alloy flowing around the periphery of said piston.

11. Apparatus for measuring the rheological characteristics of a metal or metal alloy at a specified temperature for carrying out the process according to any one of claims 1 to 10, characterised in that it comprises a die plate (1) made up of a longitudinal chamber (2) and a wall (3) comprising a ceramic die (4), a susceptor (5) for heating the die plate (1) and the slab (7) forming a mechanical support for the forces transmitted to the die plate (1) during drawing of the slab (7), means (6) for heating the susceptor (5), means for measuring the temperature of the die plate (1) and che slab (7) accommodated in the chamber (2), a preheating furnace (8) placed upstream of the die plate (1) for preheating the slab (7), means for moving the slab (7) longitudinally, from the preheating furnace (8) towards the chamber (2) of the die plate, and pressure means for deforming the slab (7) by drawing it through the die (4).

12. Apparatus according to claim 11, characterised in that the die plate (1) is made of alumina.

13. Apparatus according to claim 11, characterised in that the susceptor (5) is molybdenum-based, so as to withstand the mechanical drawing forces at temperatures exceeding 1500°C.

14. Apparatus according to claim 11, characterised in that it further comprises a controlled cooling enclosure for the material after it has been shaped by the drawing of the slab (7).

15. Apparatus according to claim 11, characterised in that the pressure displacing means consist of a piston (11) sliding in the longitudinal chamber (2) of the die plate (1) to form a compression pump.

FIG.1

FIG. 2

ISOVALEURS DE VITESSE DE DEFORMATION GENERALISEE (sec-1)

a - $0,010 < \dot{\varepsilon} < 0,050$

b - $0,050 < \dot{\varepsilon} < 0,100$

c - $0,100 < \dot{\varepsilon} < 0,500$

d - $0,500 < \dot{\varepsilon} < 1,000$

e - $1,000 < \dot{\varepsilon} < 3,000$

f - $3,000 < \dot{\varepsilon} < 5,000$

FIG.3

EP 0 489 662 B1

$$a - 0,70 < p < 0,71$$
$$b - 0,71 < p < 0,73$$
$$c - 0,73 < p < 0,75$$
$$d - 0,75 < p < 0,77$$
$$e - 0,77 < p < 0,80$$
$$f - 0,80 < p < 0,90$$

FIG. 4

FIG. 5